# EUROPEAN PATENT APPLICATION

(11) **EP 2 092 834 A1**
(43) Date of publication of application: **26.08.2009**
(21) Application number: 08151619.7
(22) Date of filing: 19.02.2008
(51) Int. Cl.: A23J 7/00, A23L 1/30, A61K 9/00, A61K 31/688, A61K 38/00, C07K 16/12

(54) **Methods and compositions of sphingolipid for preventing treating microbial infections**

(71) Applicant: Innopact B.V., 1078 VJ Amsterdam (NL)
(72) Inventor: Ekhart, Peter Frank, 1078 VJ Amsterdam (NL)
(74) Representative: Hatzmann, Martin

(57) **Abstract**

The present invention relates to a composition for the controlled or sustained release of a sphingolipid in the oral cavity of a subject, said composition comprising a sphingolipid and releasing said sphingolipid to the oral cavity at a rate of between 50 and 500 nmoles/min when masticated, chewed or sucked and a method for preventing or treating oral infections in a subject comprising using said composition.
The combined use of a sphingolipid and a histatin for treating microtial infections.

## Description

### FIELD OF THE INVENTION

The present invention relates to methods and compositions for preventing and/or treating microbial infections. In particular the present invention relates to oral compositions for preventing and treating oral infections, such as candidiasis and dental caries.

### BACKGROUND OF THE INVENTION

The present invention relates to methods of preventing an oral infection. Oral infection is characterized by an invasion and/ or multiplication of pathogenic microorganisms in the oral cavity, which may produce subsequent tissue injury. An infection may progress to overt disease through a variety of cellular and toxic mechanisms.

Due to the emergence of antibiotic resistant strains of pathogens from routine use of anti-microbial agents, infections have become significant, especially with infants and elderly subjects suffering from respectively immature and weakened immune systems.

The oral cavity is a unique environment where different mechanisms contribute to the defense against undesired proliferation of micro-organisms. One way of defense is provided by anti-microbial peptides (AMPs) that are found in saliva, the epithelium and in neutrophils. It is believed that these AMPs play a role in susceptibility for and development of caries and periodontal disease. AMPs would protect tooth structure from bacteria-induced caries either by direct killing of cariogenic micro-organisms or by prevention of bacterial biofilm formation on the tooth surface. Important classes of oral AMP are alpha-defensins HNP1-3, beta-defensins hBD1-3, LL-37, and the histatins. These combined AMPs have a broad anti-microbial spectrum. Sub-effective levels of these AMPs can occur in subjects due to age, disease such as diabetes, internal organ derangement and hypertension or genetics. At increasing age saliva production decreases together with its capacity to maintain a healthy balanced environment in the oral cavity. Trends in the levels of saliva histatins as a function of age were described by Johnson [Johnson, D.A., et al. (2000), Archives of Oral Biology, 45, 731-740] showing a 57% decrease in histatin salivary protein levels comparing 35-44 and 65-76 years old subjects. In parallel the salivary production reduced between both age groups with 30% leading to an even stronger reduction of absolute oral histatin levels. In healthy subjects an average total histatin level of 0.0017 wt% is found in parotid saliva while at an age above 65 the average level of this AMP has dropped with 58% to 0.00073 wt%.

In the case of xerostomy subjects (suffering from a dry mouth) this often leads to oral candidiasis. These subjects often use saliva substitutes to relieve their dry mouth complaints. Apart from pharmaceutical solutions to combat the development of candidiasis using for example antimycotics like fluconazole and amphotericin no effective solutions have been commercially developed based on food compositions as a prophylactic against oral infections.

AMP's play an important role in the innate defense against microbial and viral infections. Common characteristics shared between these AMP's are their usual amphipathic traits. Amphipathic molecules are mostly hydrophobic in structure, but at one end have a region that is polar or ionic (hydrophilic), the molecular mass of these AMP's ranges normally between 2 and 6 kDa. This promotes their insertion into and transmigration over the cytoplasmic membrane of the target cell, with killing of the cell as a final consequence.

It has been observed that cellular sensitivity to cationic proteins and peptides such as salivary histatins and defensins is diminished by conditions that affect the energy status of the target cell. Energy depleted conditions are common in oral biofilms and enable the colonization of the oral cavity by unwanted micro organisms like *C. albicans, S. mutants* and *P. gingivalis* partly due to their increased resistance towards AMP's.

The reduction in saliva production can lead to oral diseases like caries and periodontal diseases. Caries is a disease caused by bacteria. In 1890, W.D. Miller suggested in his "Chemico-Parasitic Theory" the hypothesis that caries is caused by oral bacteria producing acids from digestive carbohydrates, which dissolve the hydroxyapatite of the teeth. It was later confirmed in gnotobiotic rats, for example, that normal oral bacterial flora, are involved, primarily of the mutans streptococci group. These "acidogenic" species resident in the oral cavity are associated with the presence and onset of dental caries. There are seven bacterial species within the group mutans streptococci, with *Streptococci mutans* (serotype c, e, f) found in 90% of all human isolates. There is abundant evidence that the initiation of caries requires a relatively high proportion of *S*. *mutans* within dental plaque. These bacteria adhere well to the tooth surface, produce higher amounts of acid from sugars than other bacterial types, can survive better than other bacteria in an acid environment, and produce extracellular polysaccharides from sucrose. When the proportion of *S*. *mutans* in plaque is high (in the range of 2-10%), a patient is at high risk for caries. When the proportion is low (less than 0.1 %), the patient is at low risk.

Periodontal disease is not merely a disease limited to a chronic infection of the gingival tissues, but has a risk of causing circulatory system diseases such as cardiac infarct and destruction of blood vessel due to aneurysm. Periodontal disease also attracts attention as a risk factor for induction of diabetes mellitus and premature delivery.

Besides opportunistic microorganisms like the fungal pathogen *Candida albicans* can become pathogenic leading to candidosis (oral candidiasis). This is a regular infection occurring among infants during the first few weeks after birth. Normally this yeast organizes into complex biofilms which are known for their resistance against biocidal, including AMP, attack and remain in an energy depleted condition during fasting. Similar mechanisms of resistance are attributed to *S*. *mutants* residing in oral biofilms as well.

Food is normally regarded as a source of substrate for oral microorganisms. However food has also been developed to contain well known antimicrobial compounds like polyphenols, herbal extracts like eucalyptus, clary sage (*Salvia sclarea*), marjoram, rosemary, thyme, chamomile, lavender, myrrh which are active in the oral cavity. A well-known example is xylitol containing chewing gum, which can be extracted from plants and which is capable of reducing the number of *Streptococcus mutants.* Nevertheless it is known that microorganisms can become resistant against the inhibiting properties of for example xylitol. Other ingredients also presented in chewing gum as an antimicrobial compound are for example catechin, flavonoids, tannin and chlorophyll as the active ingredient. Apart from promoting a fresh breath, no prophylactic anti-infectious claims are made by manufacturers of the herbal chewing gums.

Therefore, in the view of the aforementioned deficiencies attendant with prior art methods of treating and preventing candidiasis, dental caries and periodontal diseases, it should be apparent that there still exists a need in the art for a method and or a composition for effectively combating the oral microorganisms which lead to these conditions.

### SUMMARY OF THE INVENTION

In a first aspect, the present invention provides a composition for the controlled or sustained release of a sphingolipid in the oral cavity of a subject, said composition comprising a sphingolipid and releasing said sphingolipid to the oral cavity at a rate of between 50 and 500 nmoles/min when masticated, chewed or sucked.

In one preferred embodiment, said composition is a food item or food supplement. In another preferred embodiment the said composition is a weaning product.

In another preferred embodiment said composition is a confectionery product.

In yet another preferred embodiment, said composition is of a non ingestible chewable material, preferably said composition being a saliva stimulating chewing gum.

In still a further preferred embodiment, said composition potentiates the antimicrobial effect of a salivary antimicrobial peptide (AMP). Said antimicrobial peptide is preferably a histatin, most preferably histatin 5 (DSHAKRHHGYKRKFHEKHHSHRGY).

In yet another preferred embodiment, said composition further comprises histatin 5.

In yet another preferred embodiment, said sphingolipid is a sphingoid base, preferably a sphingoid base selected from the group of sphinganines, sphingosines or phytosphingosines, preferably phytosphingosines. Another preferred sphingolipid is sphingomyelin. Sphingolipids may also be provided as a mixture of different chemical species.

In another aspect, the present invention provides a method for preventing or treating an oral infection in a subject in need of such prevention or treatment, comprising administering to the oral cavity of said subject a composition releasing a sphingolipid at a rate of between 50 and 500 nmoles/min and allowing said subject to produce saliva while maintaining said composition in contact with the saliva in said oral cavity for a sufficient period of time to allow for the potentiation by said sphingolipid of a salivary antimicrobial peptide, thereby preventing or treating said oral infection.

In a preferred embodiment of a method of the invention said antimicrobial peptide is a histatin, most preferably histatin 5.

In another preferred embodiment of a method of the invention said oral infection is selected from candidiasis (candidosis), periodontitis, dental caries, and pulpitis.

In yet another preferred embodiment of a method of the invention said sphingolipid is a sphingoid base, preferably one that is selected from the group of sphinganines, sphingosines or phytosphingosines, preferably phytosphingosines.

In yet another preferred embodiment of a method of the invention said period of time is between 15 seconds and 24 hours.

In yet another aspect, the present invention provides the use of a composition of the invention for potentiating the antimicrobial effect of salivary antimicrobial peptides in the saliva of a subject.

In yet another aspect, the present invention provides the use of a composition of the invention as an antimicrobial composition.

In yet another aspect, the present invention provides the combined use of a sphingolipid and an AMP, preferably a histatin, most preferably histatin 5 for treating microbial infections.

In yet another aspect, the present invention provides an antimicrobial composition comprising a sphingolipid and an AMP, preferably a histatin, most preferably histatin 5.

### DESCRIPTION OF THE DRAWINGS

Figure 1 shows the candidacidal effect of histatin 5 (◆) and phytosphingosine (PHS) (■) determined by the microdilution viability assay (plate assay).
Figure 2 shows PI fluorescence as a function of histatin 5 concentration at different time points (min.): t=0 (◆), t=15 (▲), t=60 (*).
Figure 3 shows PI-fluorescence as a function of PHS concentration at different time points (min.): t=0 (◆), t=15 (▲), t=60 (*).
Figure 4 shows PI fluorescence as a function of time for 6.1 µM histatin 5 preincubated with (◆) 50µM PHS, 25µM (A) PHS and without PHS (■) under energy rich conditions (NaCl).
Figure 5 shows PI-fluorescence as a function of time for 6.1 µM histatin 5 preincubated with 0µM(◆) and 25µM (■) PHS under energy depleted conditions in NaN₃.
Figure 6 shows PI-fluorescence as a function of histatin 5 concentration at t=0 preincubated with 25µM(◆) and 0µM (■) PHS under energy rich conditions in NaCl.
Figure 7 shows PI-fluorescence as a function of histatin 5 concentration at t=20 preincubated with 25µM(◆) and 0µM (■) PHS under energy rich conditions in NaCl.
Figure 8 shows PI-fluorescence as a function of histatin 5 concentration at t=0 preincubated with 25µM(◆) and 0µM (■) PHS under energy depleted conditions in NaN₃.
Figure 9 shows PI-fluorescence as a function of histatin 5 concentration at t=20 preincubated with 25µM(◆) and 0µM (■) PHS under energy depleted conditions in NaN₃.
Figure 10 shows the chemical structure of "sphingoid" or "sphingoid base", which term refers to the IUPAC nomenclature for sphinganine, [D-erythro-2-amino-1,3-octadecanediol (Figure 10.1)], to its homologs and stereoisomers (Figures 10.2 and 10.3), and to the hydroxy and unsaturated derivatives of these compounds (Figures 10.4-10.6). The term "long-chain base" may be used in a wider sense to indicate any base containing a long-chain aliphatic radical.

### DETAILED DESCRIPTION OF THE INVENTION

### Definitions

The term "controlled release", as used herein with reference to an agent means that the release of the agent occurs such that active concentrations thereof are maintained spatially or temporally within a desired concentration range. Spatial control preferably refers to maintenance of active concentrations at the site of release, while temporal control includes *inter alia* reference to "non-immediate" or delayed release and "sustained-release". Temporal control preferably refers to maintenance of active concentrations over an extended period of time. The latter form is also referred to herein as "sustained-release".

The term "sphingolipid" as used herein means a natural and synthetic substance comprising a long-chain base (LCB) (i.e. sphingoid base, a long-chain hydrocarbon material derived from d-erythro-2-amino-1,3-diol), generally comprising a polar head group, and may include reference to such compounds further comprising an amide-linked fatty acid, or to such compounds generally referred to as "lysosphingolipids" for the N -deacylated form from which the fatty acid chain bonded via an acid-amide bond to the amino group of the sphingoid has been eliminated. Preferred sphingolipids in aspects of the present invention are lysosphingolipids, most preferably sphingoid bases.

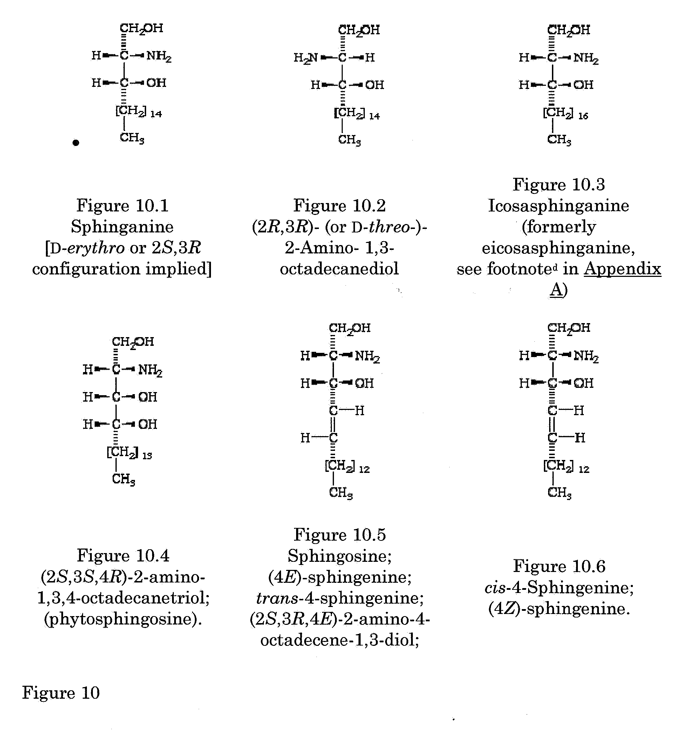

The term "sphingoid base" as used herein refers to long chain amino alcohols that may differ in length of the alkyl chain lengths and extend of branching. The most common long-chain bases in mammals are sphingosine, sphinganine and phytosphingosine (see also the chemical structures thereof and of derivatives in Fig 10).

The term "oral cavity" as used herein refers to the part of the mouth behind the teeth and gums that is bounded above by the hard and soft palates and below by the tongue and the mucous membrane. The latter connecting it with the inner part of the mandible.

The terms "masticated", "chewed" and "sucked" as used herein refer to the grinding or crushing with the teeth, molars or between palate(s) and tongue, the production of saliva, and the mixing with the saliva to prepare for swallowing and digestion.

The term "food item" as used herein refers to a food product prepared by combining two or more ingredients.

The term "food supplement" as used herein refers to a composition that can be consumed in addition to the normal food intake and which comprises elements or components that are not, or in only minor amounts, present in the normal diet and of which sufficient or increased consumption is desired. The composition of a food item does not necessarily differ much from that of a food supplement. Food supplements may be in a form wherein the composition has been compressed into tablet form, poured into capsules or powdered. A food supplement generally is a composition suitable for human consumption which comprises sphingolipids in increased concentrations or in overabundance relative to the normal food item, or is a composition which is enriched with the sphingolipid. A food supplement according to the present invention may further have other properties suitable for human consumption, such as controlled/ sustained release properties. A food supplement may also be suitable for animal consumption, referred to herein as a feed supplement.

The term "weaning food" as used herein refers to a foodstuff intended for nutrition of infants during their first 12 months of life, in particular the term refers to a food that substitutes mother's milk, breast milk or infant formula in the diet of a child or young mammal.

The term "infant formula" as used herein refers to a foodstuff intended for complete nutrition of infants during the first six months of life.

The term "infant" as used herein refers to children under the age of 12 months.

The term "potentiates" as used herein refers to the promotion and strengthening of a biochemical effect.

The term "antimicrobial effect" as used herein includes reference to biostatic activity, i.e., where the proliferation or growth of microbiological species is inhibited, reduced or eliminated, and to biocidal activity where microbiological species are killed or the viable microbial number is reduced.

The term "antimicrobial peptide" as used herein includes reference to any peptide having antimicrobial effect, in particular to salivary antimicrobial peptides.

The term "salivary antimicrobial peptide" as used herein refers to natural antibiotics found in each of the compartments of the oral cavity and periodontium. In particular, salivary antimicrobial peptide are formed in cells of the salivary glands and transported to the oral cavity by the saliva.

The term "histatin" as used herein refers to a group of (natural or synthetic) proteins or peptides with bactericidal and fungicidal activity that contribute to the innate (nonimmune) defense of the oral cavity and that are found in the saliva of mammals. Salivary histatins (Hsts), also referred to as histidine-rich polypeptides (HRPs), are homologous, structurally related histidine-rich cationic peptides. Human parotid saliva contains a total of 6 salivary histidine-rich polypeptides, referred to as Hst 1-6 or HRP 1-6, of which Hst 1, Hst 3, and Hst 5 are the most important. In vitro, Hst 5 (24 amino acids) is the most toxic to *C*. *albicans* at physiological concentrations (15 to 30 µM). Histatins 1, 3, and 5 contain 38, 32, and 24 amino acids, respectively. Histatin 2 and histatin 4 are autoproteolytic degradation products of Hst 1 and Hst 3, respectively. Histatin 2 is identical to the carboxyterminal 26 residues of histatin-1. Histatin 4 is identical to the carboxyterminal 20 residues of histatin-3. Histatin-6 is identical to histatin-5, but contains an additional carboxyterminal arginine residue. With the exception of Glu (residue 4) and Arg (residue 11) in histatin 1, the first 22 amino acid residues of histatins 1, 3 and 5 are identical, and the carboxyl-terminal 7 residues of histatins 1 and 3 are also identical. The complete sequence of histatin 5 is contained within the amino terminal 24 residues of histatin 3. The structural data suggest that histatins 1 and 3 are derived from different structural genes, whereas histatin 5 is a proteolytic product of histatin 3. All histatins have antibacterial and antifungal activity and exhibit the ability to kill the pathogenic yeast, *Candida albicans*. Histatin 5 refers to the peptide with the following amino acid sequence: DSHAKRHHGY KRKFHEKHHS HRGY. Histatin 3 refers to the unprocessed precursor protein expressed from gene *HTN3* having the amino acid sequence MKFFVFALIL ALMLSMTGAD SHAKRHHGYK RKFHEKHHSH RGYRSNYLYD N (UniProtKB/Swiss-Prot entry P15516). Histatin 1 refers to the unprocessed precursor protein expressed from gene *HTN1* having the amino acid sequence MKFFVFALVL ALMISMISAD SHEKRHHGYR RKFHEKHHSH REFPFYGDYG SNYLYDN (UniProtKB/Swiss-Prot entry P15515). Thus, the term histatin, as used herein refers to the gene products of either *HTN3* or *HTN1*, or a degradation product thereof having antibacterial and antifungal activity, more preferably having the ability to kill the pathogenic yeast, *Candida albicans*.

The term "oral infection" as used herein refers to microbial overgrowth of an undesired microorganism in the oral cavity including the enamel. Oral infections in particular include, but are not limited to candidosis (also called oral candidiasis, trush, mycotic stomatitis, moniliasisor white mouth, and also referred herein as "candidiasis"), dental caries, and periodontal disease.

The term "subject " as used herein refers to but is not limited to, mammals, including e.g. a human, non-human primate, mouse, pig, cow, goat, cat; and non-mammal animals, including e.g. a non-mammalian vertebrate, such as a bird (e.g. a chicken or a duck) or a fish, and an invertebrate.

The term "microbial" as used herein in the context of the terms "microbial infection" and "antimicrobial activity" refers to any microorganism, including bacteria, fungi, yeast and virus, in particular fungi, most preferably *Candida* spp.

### DESCRIPTION OF THE PREFERRED EMBODIMENTS

The present inventors discovered that sphingolipids are capable to potentiate the innate immune system in the oral cavity. In more detail it was found that the sphingoid base phytosphingosine can be used to potentiate the antimicrobial properties of the antimicrobial peptide histatin 5 present in the oral cavity surprisingly resulting in an increased inhibition speed of an oral pathogenic microorganisms for example the opportunistic fungi C. *albicans* under energy rich conditions. Under energy depleted conditions it was surprisingly found that the fully inhibited antimicrobial action of histatin 5 was dramatically restored by adding low concentrations of phytosphingosine.

This surprising finding may now be used to strengthen the innate oral immune system of individuals risking to develop an oral infection or to treat oral infections. The oral microbial biofilms harboring many undesired microorganisms creates unfavorable conditions for AMP's to exert their antimicrobial action. Under immuno compromised oral conditions like for example with infants and eldery people expressing sub-optimal levels of AMP these undesired microorganisms can develop into oral infections. Alternatively due to genetic disposition subjects can express sub optimal levels of anti-microbial peptides resulting in the accelerated development of caries and even pulpitis. By potentiating the activity of a subjects' oral AMP's by the use of the present invention this process can be inhibited or slowed down.

The present inventors therefore now provide a composition for the controlled or sustained release of a sphingolipid in the oral cavity of a subject, said composition comprising a sphingolipid and releasing said sphingolipid to the oral cavity at a rate of between 50 and 500 nmoles/min when masticated, chewed or sucked. The released sphingolipid potentiates the innate immune system including the AMP histatin 5 against undesired micro-organisms like *C. albicans* especially when these micro-organisms reside under energy depleted conditions like in oral biofilms.

Is has now been shown that such a composition is capable of potentiating the AMP's belonging to the innate oral immune system independent of the microorganism its energy status. This results in a higher level of defense against potential oral infections preventing their development or offering a method of treatment.

The present invention has as an additional advantage over the prior art compositions/methods that for the first time a food borne broad spectrum AMP potentiating composition has been identified offering cheap and convenient ways of prevention for groups risking oral infection. Besides in contrast to bitter tasting and pigmenting polyphenols and herbals with a distinct taste and smell, sphingolipids do not contribute significantly to the sensory profile of a food further enhancing its use in commonly commercialized food products.

Additionally the use of low levels of sphingolipid does not affect the manufacturing process of the food composition drastically also due to its stability in contrast to xylitol which can not be processed in all food matrices targeted due to restrictions in daily allowance. Sphingolipids can be formulated without undesired interference with other food ingredients also under a broad range of process conditions. The very low dosages may offer a cost effective route for preparing a functional food product as defined in Directive EC1924#2006 Article 14 and does not pose a safety problem for use in food items or food supplements.

### The composition of the invention

In one aspect of the invention the oral composition is a food product including weaning foods or confectionery. The weaning food may be an infant formula, a growing up milk, a cereal, all varieties of ready made meals, fruit preparations, drinks or desserts. The weaning food composition for improving the oral innate immune system preferably includes at least the regular nutritional ingredients formulated as known by the skilled person, and in addition to that, an effective amount of a sphingolipid resulting in a controlled release from the food product at 50 up to 500 nM of sphingolipid per minute.

The controlled release rate of 50 - 500 nM per minute as used in aspects of the present invention is based on an average saliva production of 3 ml/min, and a projected concentration of the sphingolipid of around 25 µM at the site of action.

The confectionery may be a hard candy, chewable candy, filled candy or a pressed tablet. The confectionery composition for improving the innate oral immunity includes at least one of a sugar or a sugar alcohol and an effective amount of a sphingolipid. In still another aspect of the invention the oral composition is a chewing gum or any variation including but not limited to bubble gums, pellets, gum balls or sticks. The chewing gums may be coated or not coated and be of a variety of flavors, shapes and sizes. A chewing gum composition for maintaining the innate immunity of an individual includes a water soluble bulk portion, at least one flavoring agent, a gum base portion, and an effective amount of a sphingolipid resulting in a controlled release at 50 up to 500 nM per minute.

The size of the composition is generally limited to the regularly applied portions for marketed food products. The high efficacy of the sphingolipid at low dosage levels enables the use of all food matrices envisaged for this composition. Doses for ingestion of the food or food supplement may vary in size and are not limited to the values corresponding to the recommended amounts. Herein the food or the food supplement is not intended to be limited to a specific weight or specific dose of the food or food supplement. A composition of a food or food supplement according to the invention may, in principle, have any form suitable for consumption by humans or animals. A suitable embodiment is a composition in the form of a weaning food, a candy product or a chewing gum.

### Amount of the sphingolipid in the composition

The sphingolipid is formulated in weaning foods at a level ranging between 0.00009 and 1wt% and even more preferably between 0.0001 and 0.07 wt% together with the regular ingredients used. All weight percentages as provided herein are based on the weight of the total composition, unless otherwise indicated.

For candy based compositions the dosage range is between 0.0001 and 2.5 wt% and more preferably between 0.0005 and 1 wt% together with the regular candy related ingredients.

For chewing gum compositions the dosage range for sphingolipids ranges between 0.0005 and 5wt% and even more preferably between 0.002 and 1.2 wt% together with the regular gum related ingredients.

### Suitable sphingolipids

In one embodiment, the sphingolipid is preferably a sphingoid base. The sphingoid bases of preference are sphinganine, sphingosine and phytosphinosine, a derivative thereof or a mixture of two or more of these compounds. Another preferred sphingolipid is sphingomyelin. The origin of the sphingolipid does not influence their usability for the present invention. The sphingolipid may be obtained from e.g. a natural source or from a chemical synthesis process. In principle any origin is suitable and sphingolipid may also be isolated from for instance milk, blood, meat, brains or soy for use in food or supplement preparations or method according to the invention. However it is desirable to apply a production process such that a sphingolipid is obtainable in sufficient quantities at commercially feasible prices. In that regard, some current sources of sphingolipid may have disadvantages. In case of chemically synthesis, it is very difficult to prepare the correct stereochemical configuration. In case of purification of animal and/or plant tissue extracts, the amounts of sphingolipid, and in particular of sphingoid bases are very small, making their isolation costly. Moreover, animal sources are believed to be unsafe due to the presence of viruses and other infectious agents, such as the agent causing BSE (mad cow's disease). Therefore, sphingolipids are preferably obtained from a microbial fermentation process. More preferably, they are obtained from a yeast, especially preferably from *Pichia ciferrii*. Yeast derived phytosphingosine is human tissue-identical, as it is reported to have the same stereochemical configuration as mammalian phytosphingosine, i.e. the D-D-erythro configuration.

The sphingoid base of preference, phytosphingosine, may for example be obtained from Degussa, Düsseldorf, Germany. The product is obtained from a yeast *Pychia ciferrii* and is provided as a powder. The phytosphingosine may be dissolved in ethanol or a suitable hydrophobic food component of the food product of interest and may be warmed prior to making the composition.

### Suitable antimicrobial peptides

Antimicrobial peptides (AMPs) suitable for use in aspects of the present invention are generally those that are naturally present in saliva. Salivary antimicrobial peptides include, but are not limited to alpha-defensins HNP1-3, beta-defensins hBD1-3, LL-37, and the histatins. The histatin is preferably histatin 1, 3 or 5, or a fragment thereof, or combinations thereof, preferably histatin 5. As the antimicrobial peptide, one may use the natural peptide or protein. Alternatively, in compositions comprising additional AMPs, the AMP may be a synthetic peptide or protein. The peptide is generally the native peptide having the antimicrobial activity. Alternatively one may use an antimicrobially active fragment of the peptide, such as for instance Dh5 (11-24) with peptide sequence KRKFHEKHHSHRGY or P-113 (4-15) with peptide sequence AKRHHGYKRKFH, which sequences are fragments derived from histatin 5. can be used to design synergistic compositions of antimicrobial peptides and sphingolipids. Also the efficacy of other AMPs, such as alfa and beta defensins, is sensitive to energy depletion. Hence the potentiation of such AMPs is envisaged in aspects of the present invention. The AMP is preferably present in an antimicrobial effective amount,. The skilled person will be able to determine at which concentration the AMP has its optimum antimicrobial amount, in the absence of any substantial cytotoxic effects. The amount will depend on the production rate of the peptide by the salivary glands and its resulting concentration in the saliva, or on its release rate from the composition of the invention, both of which may be determined by methods well known in the art.

### Controlled or sustained release formula

The controlled/sustained release of the sphingolipid may be realized by incorporating it in a slowly dissolving matrix such as a sugar matrix in candy products or alternatively by encapsulating the active ingredient in a slowly dissolving starch based matrix, capable of being degraded by the amylase enzymes in saliva thereby releasing the encapsulated sphingolipid. Alternatively the exposure to an effective dosage of sphingolipid can be obtained by exposing the oral cavity multiple times to a smaller unit of a rapidly dissolving composition like for example small size candy tablets. In this way a controlled release formula comprises a set of multiple units of a single dosage formula, which when provided orally in succession, provide for the desired sustained release effect and an effective concentration of the sphingolipid in the oral cavity. The term effective dosage of a sphingolipid in the context of the present invention refers to an amount of sphingolipid capable of being released at a rate from the composition of the present invention that results in a concentration (the effective concentration) of the sphingolipid at the desired locality that provides for a potentiating effect on the antimicrobial effect of the AMP present at that same locality.

In the case of a weaning formula like a infant milk formula controlled release of the sphingolipid may be realized by dipping a wetted teat into the infant formula powder and to present it to the infant. Alternatively a starch coated teat is constructed carrying an starch encapsulated sphingolipid coating resulting in a controlled release of the active composition and resulting in a candy product for infants.

### Additional ingredients

To a food and food supplement comprising a sphingolipid which improve for instance, the nutritional profile, texture, taste or smell may be added. For instance a food according to the invention may also comprise sources of protein, carbohydrate and fat, as well as vitamins, minerals, electrolytes, trace elements and other suitable addition, such that the food may be used in the form of a food supplement with nutritional constituents. As a source of protein, in principle any protein suitable for use in food formulations and mixtures thereof may be used in a nutritional supplement according to the invention. Such proteins comprise for instance animal protein, such as whey protein, whey protein concentrate, whey powder, egg protein, egg albumen, casein or milk albumin, and vegetable protein, such as soy protein, soybean meal or protein from soymilk. For the choice of the protein source, the biological value of the protein can be an important criterion, with for instance caseinate including calcium caseinate, but also whey, milk albumin, egg albumin and whole egg proteins being among the proteins with the highest biological value, because they contain high content essential amino acids.

Suitable carbohydrates for use in a nutritional supplement according to the invention comprise for instance, simple short chain carbohydrates such as mono and disaccharides, or a combination thereof. A sugar may be chosen because of desired organoleptic properties. A complex polysaccharide may for instance, be suitably used as a dietary fiber or a slow release matrix. For food and food supplements also sugar polyols and synthetic sweeteners such as saccharides, cyclamates, aspartamine, aspartame, acesulfame K and-or sorbitol may be formulated in order to reduce their cariogenicity and caloric content.

As fats, in principle, all possible fats and oils suitable for consumption may be used. Vitamins and minerals may for instance, be added to the food or food supplement in accordance with applicable rules of health authorities and may comprise all vitamins and minerals recommended by these bodies, such as vitamins A, B1, B2, B12, folic acid, niacin, panthotenic acid, biotin, C, D, E and K. As minerals for instance, iron, zinc, iodine, calcium, magnesium, chrome and selenium may be added.

Electrolytes such as sodium, potassium and chlorides, and trace elements and other additions may also be comprised in a food or food supplement according to the invention and are, if present therein, preferably used in the amounts recommended for these substances. A food or food supplement according to the invention may further comprise constituents such as texture/improving constituents, colorings, aromatic substances, flavorings, spices, fillers, emulsifiers, stabilizers, preservatives, antioxidants, dietary fibers, and other nutritional supplements such as amino acids, choline, lecithin, fatty acids, etc. The choice for such constituents which can be added are known to a skilled person, while the choice may, for instance, be guided by the recommended daily amounts for children and adults.

Emulsifiers may be added for stability of the final product. Examples of suitable emulsifiers comprise, for instance, lecithin (e.g. egg or soy derived) and-or mono and diglycerides. As stabilizers, for instance, carob, guar and carrageen gum may be used.

Preservatives may also be added to prolong the storage life of the product. Preferably, preservatives such as potassium sorbate, sodium sorbate, potassium benzoate, sodium benzoate or calcium disodium EDTA are used.

### Preparation of the compositions of the invention

In one embodiment of the invention the food product is a chewing gum which is manufactured from traditional coherent gum. In this manner the sphingolipid and optionally other ingredients are mixed into the gum base mass. The mixing operation may take place at elevated temperature to decrease the viscosity of the chewing gum formulation thereby facilitating the mixing. After the mixing the chewing gum formulation is normally sent through rollers to form sheets of chewing gum from which pieces of chewing gum are punched or scored out. In general traditional coherent chewing gum is manufactured by sequentially adding the various chewing gum ingredients to a commercially available mixer known in the art. After the ingredients have been thoroughly mixed, the gum mass is discharged from the mixer and shaped into the desired form such as by rolling into sheets and cutting into sticks, extruding into chunks or casting into pellets. Alternatively, coherent chewing gum may be manufactured by extrusion. Generally the ingredients are mixed by initially melt the gum base and feed it to the running mixer. The base may also be melted in the mixer itself. Color or emulsifiers may also be added at this time. A softening agent such as glycerin may also be added at this time, along with syrup and a portion of the bulking agent. Further portions of the bulking agent may be then be added to the mixer. A flavoring agent is typically added with the final portion of the bulking agent. It will be recognized to those skilled in the art that variations of the above-described procedure may be used. In another embodiment the chewing gum is manufactured from compressed granules. Thus the gum base is present as granules and is mixed with the active agents which may also be present as granules or powder and optionally other ingredients. The mixture is filled into a press that presses the mixture to form compressed chewing gum tablets. Use of granules is particular advantageously when one or more of the active ingredients are sensitive towards elevated temperatures as the mixing and pressing can be done at low temperatures, e.g. normal room temperature.

Compressed chewing gum may in some embodiments preferably be mixed with flavorizing agents (flavors) and/or sweeteners. Moreover the chewing gum according to the invention may be centre filled gum (centre filled with liquid, gel or powder), coated gum or gum formed as sticks. Preferably the gum has an average weight of about 0.5-5 gram, preferably from 1.2 to 3.5 gram. In a preferred embodiment of the chewing gum according to the invention the chewing gum is layered. The chewing gum may comprise two or three or more layers. The layers may be placed on top of each other or side by side. Optionally the layers have different colors. When the chewing gum is layered it is possible to provide embodiments wherein different active ingredients are present in different layers in the chewing gum.

In some embodiments of the invention it is preferred that the chewing gum is coated. A coating may protect the active agents from decomposition e.g. caused by oxygen. Moreover coating may contribute to maintain desired moisture content in the chewing gum or other physical conditions to avoid breakdown of an active ingredient. The coating may be a hard coating or a film coating. In an embodiment of the invention the chewing gum is consequently coated with an outer coating. When the chewing gum has a coating at least one active agent may be present in the coating. Such an embodiment can for instance be advantageously when rapid release of one or more active agents is desirable. In a preferred embodiment of the invention the hard coating is selected from the group consisting of a sugar coating and a sugarless coating and a combination thereof. In a further embodiment of the invention the hard coating comprises 50 to 100% by weight of a polyol typically selected from the group consisting of sorbitol, maltitol, mannitol, xylitol, erythritol, lactitol and isomalt. In an alternative embodiment of the invention the outer coating is an edible film comprising at least one component selected from the group consisting of an edible film forming agent and a wax. In a preferred embodiment of the invention the film-forming agent is selected from the group consisting of a cellulose derivative, a modified starch, a dextrin, gelatin, shellac, gum Arabic, zein, a vegetable gum, a synthetic polymer and any combination thereof. In an embodiment of the invention the outer coating comprises at least one additive component selected from the group consisting of a binding agent, a moisture absorbing component, a film forming agent, a dispersing agent, an anti sticking component, a bulking agent, a flavoring agent, a lipid component like for example a sphingolipid, an antimicrobial peptide, a sugar, an acid.

In one embodiment the outer coating is a soft coating comprising a sugar free coating agent. The invention also encompasses an embodiment in which the chewing gum comprises at least one barrier layer. A barrier layer may serve to separate two active agents that will react when mixed. Optionally the barrier layer is a layer in a layered tablet e.g. a chewing gum tablet comprising three or more layers. According to the invention it is preferred that one or more sphingolipids constitutes from 0.0005% up to 5% of the chewing gum. Preferably the sphingolipid composition constitutes 0.002 up to 1.2%, more preferred 0.05-0.5% of the chewing gum. Preferably the one or more fresh breath agents constitute 0.01% to 20% more preferred 0.02-8% of the chewing gum. Preferably the one or more whitening agents constitute 0.01% to 20% more preferred 0.03-12% of the chewing gum. Preferably the one or more anti microbial peptide agents constitutes 0.01% to 20% more preferred 0.02-1% of the chewing gum. Preferably the one or more anti plaque agents constitutes 0.03% to 50% more preferred 0.05-35% of the chewing gum.

The infant formula according to the present invention preferably comprises a protein source in an amount of preferably not more than 2.0g/100kcal, more preferably 1.8 to 2.0/100kcal. The type of protein is not believed to be critical to the present invention provided that the minimum requirements for essential amino acid content are met and satisfactory growth is ensured although it is preferred that over 50% by weight of the protein source is whey. Thus, protein source based on whey, casein and mixtures thereof may be used as well as protein sources based on soy. As far as whey proteins are concerned, the protein source may be based on acid whey or sweet whey or mixtures thereof and may include alpha-lactalbumin and betalactoglobulin in whatever proportions are desired.

The proteins may be intact or hydrolysed or a mixture of intact and hydrolysed proteins. It may be desirable to supply partially hydrolysed proteins (degree of hydrolysis for instance between 2 and 20%), for example for infants believed to be at risk of developing cows' milk allergy. If hydrolysed proteins are required, the hydrolysis process may be carried out as desired and as is known in the art. The infant formula according to the present invention contains a source of lipids. The lipid source may be any lipid or fat which is suitable for use in infant formulas. Preferred fat sources include palm olein, high oleic sunflower oil and high oleic safflower oil. The essential fatty acids linoleic and alfa-linolenic acid may also be added as may small amounts of oils containing high quantities of preformed arachidonic acid and docosahexanoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula may further contain vitamins and minerals understood to be essential in the daily diet and in nutritionally significant amounts, meaning that per 100 grams at least 15% of the recommended daily dosage for the intended user are provided. Minimum requirements have been established for certain vitamins and minerals. Examples of minerals, vitamins and other nutrients optionally present in the infant formula include vitamin A, vitamin B1, B2, B6, B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary the infant formula may contain emulsifiers and stabilizers such as soy lecithin, citric acid esters of mono- and di-glycerides, and the like. This is especially the case if the formula is provided in liquid form.

The infant formula may optionally contain other substances which may have a beneficial effect such as fibers, AMP's like lactoferrin, alpha-defensins HNP1-3, beta-defensins hBD1-3, LL-37, histatins, nucleotides, nucleosides and the like. The sphingolipid composition may suitably be composed from phytosphingosine, sphingosine and sphinganine, optionally combination with sphingolipids added as a component of another ingredient like soy derived lipids. According to the invention it is preferred that one or more sphingolipids constitutes from 0.00009% up to 1% of the infant formula. Preferably the sphingolipid composition constitutes 0.0001 up to 0.07%, more preferred 0.001-0.05% of the infant formula.

The infant formula may be prepared in any suitable manner. For example, an infant formula may be prepared by blending together the protein source, the carbohydrate source, and the fat source in appropriate proportions. If used, the emulsifiers may be included in the blend. The vitamins and minerals may be added at this point but are usually added later to avoid thermal degradation. Any lipophilic vitamins, emulsifiers, sphingolipids and the like may be dissolved into the fat source prior to blending. Water, preferably water which has been subjected to reverse osmosis, may then be mixed in to form a liquid mixture. The mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchangers; for example a plate heat exchanger. The liquid mixture may then be cooled to about 60°C to about 85°C; for example by flash cooling. The liquid mixture may then be homogenized; for example in two stages at about 7 MPa to about 40 MPa in the first stage and about 2 MPa to about 14 MPa in the second stage. The homogenized mixture may then further be cooled to add any heat sensitive components; such as vitamins and minerals. The pH and the solids content of the homogenized mixture is conveniently standardized at this point. The homogenized mixture is transferred to a suitable drying apparatus such as a spray drier or freeze drier and converted to powder. The powder should have a moisture content of less than about 5% by weight.

The food product or food supplement composition of the present composition may further comprise a chewable candy base. Suitable chewable candy bases are well known in the art. In one embodiment the chewable candy base is a taffy candy base comprising a mixture of flavoring agents and cooked sugar. In one embodiment a taffy base is made by heating sugar to a temperature of about 121°C - 124°C, allowing the mixture to cool somewhat and then adding the flavoring agent and the sphingoid composition. In another embodiment the chewable candy base is a caramel candy base comprising a mixture of flavoring agent and a cooked mixture of sugar and lipids. In one embodiment, a caramel base is made by heating a mixture of sugar and lipid including the sphingoid composition to a temperature of about 115°C-124°C allowing a mixture to cool somewhat and then adding the flavoring agent.

Suitable sugars are known and include for example corn syrup, cane sugar, beet sugar, glucose. Suitable lipids are known and include butter, cream, condensed milk and vegetable oils, such as partially hydrogenated soybean oil. Suitable flavorings are known and include natural flavors, such as vanilla extract, cocoa powder, natural chocolate flavor and fruit concentrates as well as artificial flavors such as vanillin and artificial fruit flavors. Optionally the chewable candy base comprises an effective amount of a softening agent such as for example glycerin, glyceryl monostearate or a lipid component including sphingolipid compositions. The term effective amount in the present context refers to an amount that ensures that the final product is soft or flexible, i.e. not hard. The candy base composition may optionally contain other ingredients generally recognised as safe for food additive use including for example starches, such as wheat flour and corn starch, preservatives, such as butylated hydroxytoluene and butylated hydroxyanisole, food grade emulsifiers such as lecithin, propylene and glycol esters, seasonings, such as table salt and food grade colorants such as color additives that are certified or certifiable by competent authorities for food use such as for example FD&C Blue No. 1 (Brilliant Blue), FD&C Blue No.2 (Indigotine), FD&C Green No. 3 (Fast Green FCF). The soft chews of the present invention are made by combining the sphingolipid composition as well as any optional components with the chewable candy base in the desired relative amounts, mixing the components according to known methods to produce a substantially homogeneous mixture and forming the resulting mixture into individual soft chews.

According to the invention it is preferred that one or more sphingolipids constitutes from 0.0001% up to 2.5% of the candy formula. Preferably the sphingolipid composition constitutes 0.0005 up to 1.5%, more preferred 0.0005-1% of the candy formula.

Alternatively, the composition of the present invention may be provided in the form of a coating on a pacifier or silicone nipple. The invention provides the coated pacifier as well as a coating formula for coating a pacifier. The pacifier may for instance be coated by optionally dissolving the (dry) coating composition of the present invention in an aqueous solution and dipping the pacifier in the coating solution. Thereafter, the pacifier may optionally be dried to harden the coating solution, thereby providing for a sustained release formula on the outside of the pacifier that, when taken in the mouth by the suckling, will dissolve and release the active compounds.

### Determining controlled release rates

For analysis of the release rate of sphingolipid from a food product like a chewing gum or a chewable candy containing sphingolipid a specified amount of chewable candy or chewing gum for example 3 gram is chewed and masticated by at least 6 subjects for a specified time window dedicated to the food product of interest. For a chewable candy complete bolus samples are taken multiple times at least 3 times for every 20 seconds In the case of chewing gum a complete gum bolus is collected from subjects at different time intervals e.g. at 0, 5, 10 and 20 minutes. The bolus is dissolved in a suitable solvent for example chloroform for chewing gum.

For other confectionery products not incorporating a gum base or weaning formula products a mixture containing 93% chloroform: methanol (1:2 (v/v) and 7% 2M ammonium chloride solution can be used. A 4 % (w/v) solution of the confectionery product or the weaning formula product is prepared with the aforementioned solvent, which is subsequently extensively mixed on a vortex for at least 2 minutes. The mixture is incubated at 37°C for 1 hr in a closed screw tube and cooled down to room temperature. Subsequently alkaline water (0.1 ml 2M ammoniumchloride in 250 ml deionized water, pH=8-10 prepared daily) and chloroform are added (3 parts + 1.5 part) and the mixture is centrifuged for 20 minutes at 3000 rpm to obtain phase separation. After centrifugation the aqueous phase is removed and alkaline water is added to the bottom phase (again 3 parts to 1.5 part residue). After mixing and centrifugation the aqueous rinse is repeated once more. Finally the chloroform phase is dried under nitrogen and the pallet can be stored at 4°C overnight or at -20 for longer times.

The confectionery or weaning formula extracts are dissolved in 250 µl mobile phase by mixing on a vortex for 1 minute. Subsequently 50µl of orthophthalaldehyde (OPA) reagent is added and the mixture is mixed for 30 seconds on a vortex, then filtered by centrifugation through a filter of 0.45-µm pore size (in 1 minute) and left for 1 hour at room temperature before injection.

For the high performance liquid chromatography (HPLC) conditions a prefiltered and degassed mixture of methanol:water (9:1) (v/v) is used as the mobile phase. A flow rate of 2 ml/min. is used. Detection is for example done by use of a fluorescence detector at an excitation wavelength of 340 nm and an emission wavelength of 435 nm.

The formula containing sphingomyelin are dissolved at 10 mg quantities in 0.5ml aqueous methanolic hydrochloric acid. The methanolic hydrochloric acid solution is prepared immediately for use by mixing 8.6ml 12M hydrochloric acid (HCL) with 9.4ml deionized water and bringing it to a 100 ml volume with methanol. The tube containing the sphingomyelin formula is capped with a Teflon cap and left at 68°C for 15 hr. After cooling to room temperature 0.5ml saturated methanol potassium hydroxide (KOH), 0.5 ml alkaline water, 0.1 ml 2M ammoniumhydroxide and 0.6ml chloroform are added and the mixture is stirred and centrifuged for 20 minutes at 3000 rpm. The aqueous phase is subsequently discarded and the chloroform phase is rinsed three times with alkaline water and then dried under nitrogen and stored at -20°C until analysis.

Subsequently the samples can be analyzed for sphingolipid by RP-HPLC. The percentage of sphingolipid retained can be calculated based on the HPLC analysis results. By subtracting sphingoid levels found in the bolus from starting quantity presented to the subject the amount of sphingolipid released into the oral cavity as a function of time can be determined.

Sphingolipids are analyzed using RP-HPLC using an external reference curve prepared with the relevant fully specified reference sphingolipid.

### Methods for preventing or treating oral infection using the inventive compositions

The present invention provides a method to potentiate the compromised innate oral immune system of a subject including but not limited to groups at risk like infants, elderly people with reduced levels of saliva and/ or reduced levels of AMP's in their saliva, denture wearing subjects, subjects suffering from xerostomia and hospitalized people taking broad spectrum antibiotics, immunosuppressive drugs, endocrine dysfunction, bone marrow depression, nutritional deficiencies and radiation treatment. This method comprises administering to this subject a food comprising a sphingolipid which sphingolipid is suitably chosen from the group of sphingomyelin, phytosphingosine, sphingosine or sphinganine and in which food this lipid is overabundant. The term overabundant or overabundance relates to a content of a constituent in a composition which is higher than would naturally or normally or without human intervention (by enrichment) be present in such a composition or would be found therein. The overabundance or enrichment of a constituent can be the result of the specific addition of a constituent to a composition which does not normally comprise this constituent like for example a chewing or bubble gum or a (starch) coated weaning teat. This is for example realized by an enrichment of this composition with this constituent. Overabundance of a constituent may also be the result of a specific addition of a constituent to a composition which normally already comprises this constituent, but whose concentration or content is increased by the addition of values which are normally not present in such a composition; this also involves enrichment of the composition with the constituent. Because contents of sphingolipids such as phytosphingosine, sphingosine or sphinganine are normally very low in different foods, enrichment will involve dosage ranges as suggested for the present invention. In a food according to the invention, one or more sphingolipids chosen from the group of phytosphingosine, sphingosine or sphinganine are, in any case, used in an effective amount to have a growth inhibiting or killing activity against the desired microorganism in the oral cavity including for example *Candida albicans, Streptococcus mutants* and *Porphyromonas gingivalis*. Preferably the food item or food supplement contains one or more sphingolipids at an amount between 0.000009 and 5 wt%, more preferably of 0.00001 and 1-2 wt% formulated in a matrix allowing for slow release of the sphingolipid into the oral cavity. The slow release results in an active level of sphingolipid in saliva of at least 0.0006 wt% more preferably of at least 0.0008 wt% sphingolipid and maximally 0.02 wt% more preferably maximally 0.008 wt%.

The subject at risk for developing an oral infection may be administered a food item or food supplement at high or lower frequency depending on the level of risk. For a subject at high risk for developing oral infection like candidiasis, periodontitis and pulpitis or even systemic infection as a result of candidiasis or pulpitis a frequency of consumption is suggested of three times a day or more preferably six times a day independent upon the exact composition of the food item or food supplement. For subjects exposed to lower risk for developing oral infection like for example caries a single serving of a composition per week or more preferable every two days or even more preferably every day would suffice in order to maintain the number of colony forming units of pathogenic micro-organisms at safe levels.

### Circumstances of synergistic activity.

The active compounds can be delivered or released into the oral cavity for effective preventive treatment against oral infections or treatment of oral infections. Preferably the food item or food supplement contains a sphingolipid at an amount between 0.000009 and 5 wt%, more preferably of 0.00001 and 1.2 wt% formulated in a matrix allowing for slow release of the sphingolipid into the oral cavity. The period of time for release of the active composition ranges between 15 seconds up to five minutes for chewable confectionery like liquorice, fruit gums or other chewable candy and 15 seconds up to 24 hours for weaning teats, chewing gums or bubble gums. More preferably the period for release of the active composition ranges between 15 seconds and 10 minutes for all food items and food supplements envisaged by the invention.

Synergistic effects of the addition of sphingolipids and salivary antimicrobial peptides are provided when antimicrobial peptides are sufficiently detectable in saliva or otherwise supplemented to saliva together with the sphingolipid or a combination of sphingolipids. A detectable naturally abundant level of antimicrobial peptides in saliva ranges between 0.00003 up to 0.015 wt% (for AMPs in a Mw range of 2-6 kDa). Slow release of sphingolipid from a food item or food supplement applied into the oral cavity results in an active level of sphingolipid in saliva of at least 0.0005 wt% more preferably of at least 0.0006 wt% sphingolipid and maximally 0.02 wt% more preferably maximally 0.008 wt%. Optionally in another embodiment of the invention preferably when antimicrobial peptide levels are below 0.002 wt% the sphingolipid is synergistically formulated together with an anti microbial peptide composition for example histatin 5 or one or more of its equivalents like histatin 1 and 3 or one or more of its isolated active peptide sequences. In one embodiment of the invention the anti microbial peptide composition or more specifically a histatin composition is released at levels ranging between 0.00001 and 0.1 wt% in saliva more preferably at levels between 0.0002 and 0.015 wt% in saliva together with the sphingolipid dosed at levels between 0.0005 and 0.02 wt% more preferably between 0.0006 and 0.008 wt%.

### EXAMPLES

### Example 1. Effect of Hst5 and phytosphingosine (PHS) on viability and membrane integrity of C. albicans

*Growth conditions - C. albicans* (ATTC 10231), cultured aerobically at 30 °C on Sabouraud dextrose agar plates (SDA, Oxoid, Hampshire, UK) was suspended in 25 ml of Sabouraud dextrose broth in a 100 ml erlenmeyer flask. After 20 h of incubation at 30 °C, 1 ml from this suspension was sub-cultured for 1-2 h in 20 ml of Sabouraud dextrose broth, to obtain a mid-log phase culture. Cells were washed twice in 1 mM potassium phosphate (PPB) or 5 mM Tris (pH 7.2), and resuspended in the same buffer to a cell density of 2 McFarland (McF) (approximately 10⁷ cells/ml).

*Preparation of histatin 5-* The antimicrobial peptide histatin 5 (Hst5) was manufactured by solid phase peptide synthesis using Fmoc (fluoren-9-ylmethoxycarbonyl)-chemistry with a MilliGen 9050 peptide synthesizer (Milligen-Biosearch, Bedford, MA) according to the manufacturer's procedures. The following peptide sequence was prepared DSHAKRHHGYKRKFHEKHHSHRGY. N-α-Fmoc protected amino acids and preloaded PEG-PS supports were obtained from Applied Biosystems (Foster City, CA). The peptide was purified by RP-HPLC (Jasco Corporation, Tokyo, Japan) to a purity of at least 90%. For this purpose the peptides were dissolved in 0.1% trifluoroacetic acid and applied on a VYDAC C18-column (218TP, 1.0cmx25cm, 10µm particles, Hesperia, CA), equilibrated in 0.1% trifluoroacetic acid. Elution was performed with a linear gradient, from 15 to 55% acetonitrile containing 0.1% trifluoroecetic acid in 20 min. at a flow rate of 4 ml/min. The absorbance of the effluent was monitored at 214 nm and peak fractions were pooled, lyophilized and reanalyzed by RP-HPLC.

The authenticity of the peptide was confirmed by ion trap mass spectrometry with a LCQ Deca XP (Thermo Finnigan, San Jose, CA).

### Determination of the membrane-disruptive activity of histatin 5 and PHS (PI assay)

Membrane-disruptive activity of Hst5 and phytosphingosine (PHS) was determined by monitoring the fluorescence enhancement of propidium iodide (Invitrogen, Breda, The Netherlands) in Hst5- or PHS treated cells. The membrane impermeant propidium iodide (PI) only enters membrane-compromised cells, after which the fluorescence of this probe is enhanced 20-30-fold due to its binding to nucleic acids.

Cells obtained by the method described above were suspended in 1 mM PPB buffer (pH 7.2) supplemented with PI (final concentration 10 µM) and subsequently added to two-fold serial dilutions of Hst5 (50µM down to 0.045µM) or PHS (500 down to 0.5µM). Final cell density in the assay was 1 McF. PI fluorescence was monitored at 0, 15 min, and 1 h, at excitation and emission wavelengths of 544 and 620 nm, respectively, in a Fluostar Galaxy microplate fluorimeter (BMFG Labtechnologies, Offenburg, Germany). Afterwards, 50 µl from selected wells was diluted 200 times in PBS, and the numbers of survived cells were determined by plating 25 µl of these suspensions on SDA and counting the colony forming units (CFUs) after 48 h of incubation at 30 °C. All experiments were repeated at least three times in duplo.

The effects of PHS and Hst5 on the viability of *C*. *albicans* were determined in a microdilution viability assay. 50 µl aliquots from a mid-log phase culture of *C*. *albicans* were incubated with equal volumes of either peptide or PHS solutions (0.3 to 100 µM) and incubated at 30 °C for 60 min. The incubation mixtures were appropriately diluted (200- to 500-fold) in PBS and 25 µl aliquots were plated on SDA. After 48 h incubation at 30 °C the numbers of CFUs were counted. All experiments were repeated at least three times in duplo.

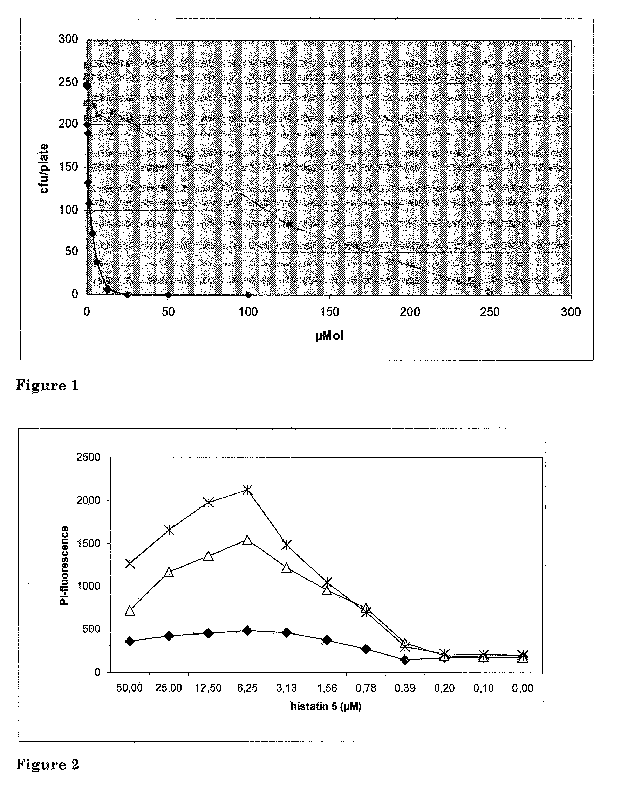

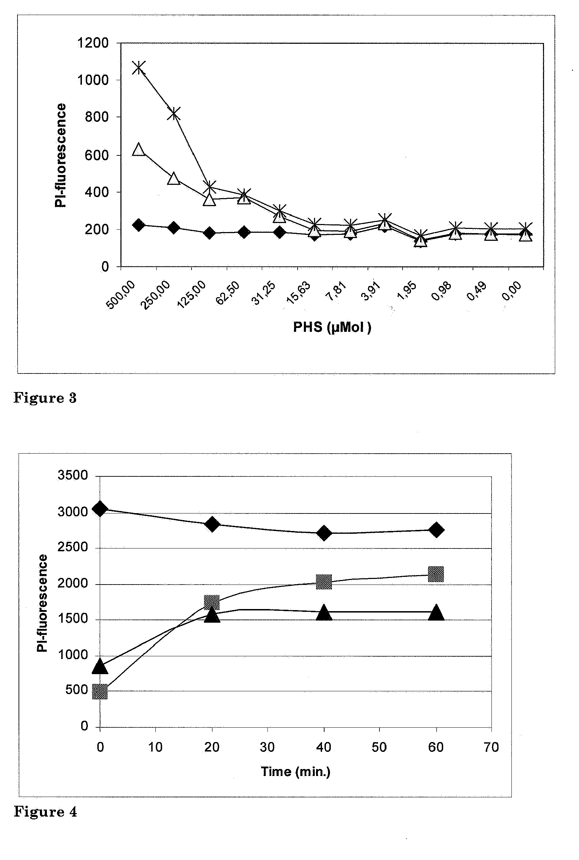

The effect of PHS-treatment on the viability of *C*. *albicans* was determined in a micro-dilution viability assay, at the concentration range 0-500µM (Fig. 1). For comparison the effect of treatment with Hst5 (0-50µM) is shown. In 1 mM PPB complete killing by Hst5 was already reached at 10 µM, whereas PHS-mediated killing occurred at a much higher concentration (250 µM). Similar results were obtained in the PI-assay, i.e. Hst5 induced the uptake of PI at much lower concentrations (fig 2) than PHS (Fig 3) after 15 and 60 minutes.

### Example 2. Effect of phytosphingosine (PHS) on Hst5 challenged viability and membrane integrity of C. albicans

### Synergism between Histatin 5 and PHS: effect of energy depletion.

Cells were suspended in 5 mM Tris buffer (pH 7.2), supplemented with either 5 mM NaCl or 5 mM NaN₃. These suspensions were subsequently incubated with 0, 10, 50 and 100 µM PHS dissolved in 5 mM Tris buffer. After 60 minutes of incubation at 30 °C, PI was added, and subsequently dilution series of Hst 5 in the corresponding buffer were added. Final concentration of PI was 10 µM, final concentrations of Hst5 ranged between 0 and 50 µM, final cell density was 10⁷ cells/ml and final PHS concentrations were 0, 5, 25 and 50 µM. After 0, 20, 40 and 60 minutes the PI fluorescence was monitored at excitation and emission wavelengths of 544 and 620 nm, respectively, in a Fluostar Galaxy microplate fluorimeter (BMFG Labtechnologies, Offenburg, Germany).

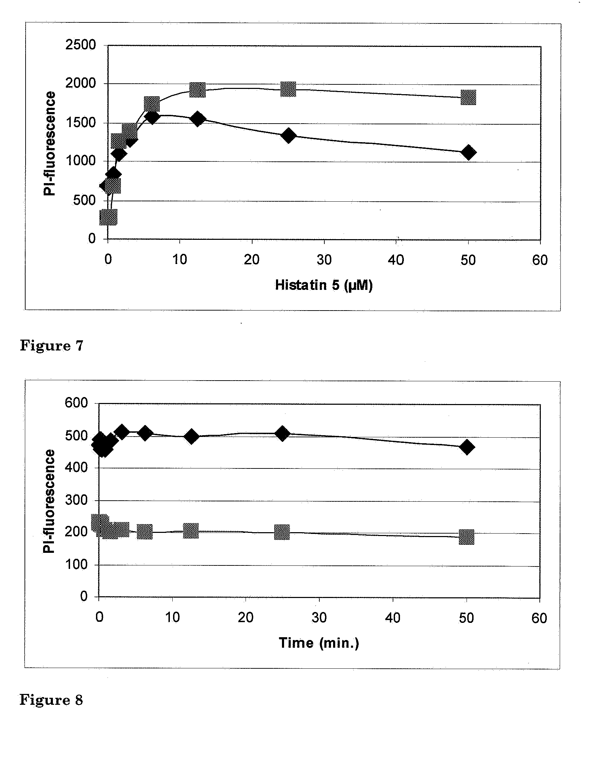

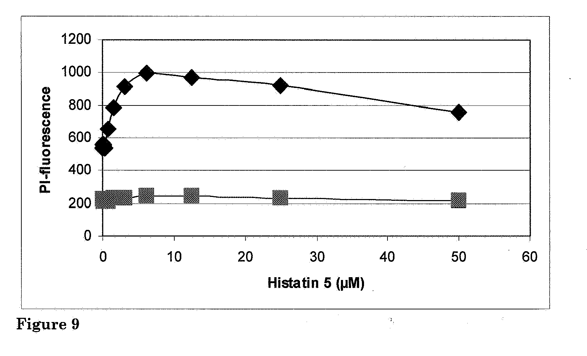

This experiment revealed that energy depletion does protect *C*. *albicans* against Hst5, but not against PHS (See Fig. 8 t=0 min. and Fig. 9 t=20 min.). Preincubation with 25 or 50µM PHS in the presence of NaCl instantaneously accelerates the Hst5-mediated influx of PI, relative to the control, preincubated with 0 µM PHS (Fig. 4, squares, 6.1 µM Hst5).

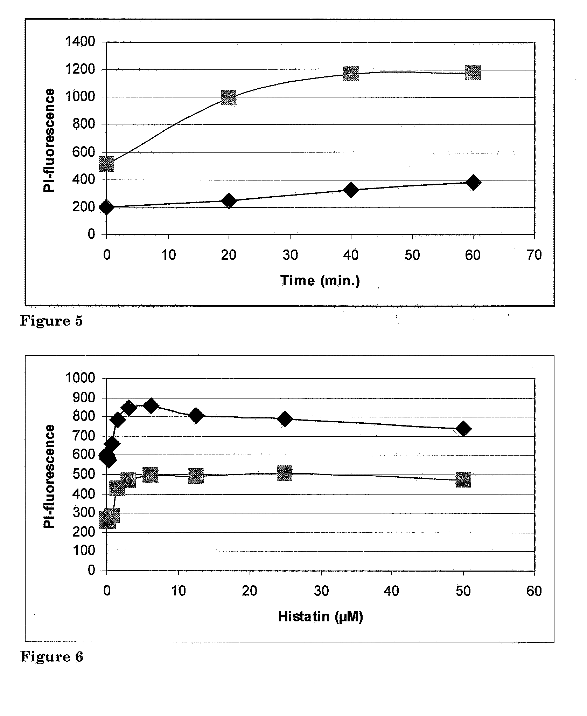

After pre-incubation with 25µM PHS, immediately an additional increase in the PI fluorescence intensity is found at Hst5 concentrations as low as 0.75µM (Fig.6).

The Hst5 induced PI-fluorescence in PHS preincubated cells reached its maximal value after 40 to 60 minutes (fig 5) which is comparable to the controls, not preincubated with PHS (Fig. 4, squares).

Preincubation with 25 µM PHS in the presence of NaN₃ induces an increase in PI fluorescence, comparable to that of the control (NaCl instead of NaN₃ fig 6). Subsequent addition of Hst5 increases the PI-fluorescence in a Hst5 dose-dependent way. This becomes manifest after 20 minutes incubation with Hst5 (Fig 9 diamonds), and reaches its maximum after approximately 40 minutes (Fig 5 squares). The extent of this additional increase is approx 500 fluorescence units which is smaller than 1000 fluorescence units for its control (NaCl instead of NaN₃ fig. 7).

Pretreatment of *C. albicans* with non lethal dosages PHS surprisingly increases its sensitivity to Hst5, in particular under energy-depleted conditions, which normally render *C. albicans* insensitive to Hst5.

## Claims

1. A composition for the controlled or sustained release of a sphingolipid in the oral cavity of a subject, said composition comprising a sphingolipid and releasing said sphingolipid to the oral cavity at a rate of between 50 and 500 nmoles/min when masticated, chewed or sucked.

2. Composition according to claim 1, wherein said composition is a food item or food supplement.

3. Composition according to claim 1, wherein said composition is a confectionery product.

4. Composition according to claim 1, wherein said composition is a weaning food product.

5. Composition according to claim 1, wherein said composition is of a noningestible chewable material, preferably said composition being a saliva stimulating chewing gum.

6. Composition according to any one of the preceding claims, wherein said composition potentiates the antimicrobial effect of a salivary antimicrobial peptide.

7. Composition according to claim 6, wherein said antimicrobial peptide is a histatin, preferably histatin 5.

8. Composition according to any one of the preceding claims, further comprising an antimicrobial peptide, preferably a histatin, more preferably histatin 5.

9. Composition according to any one of the preceding claims, wherein said sphingolipid is a sphingoid base or a sphingomyelin, preferably a sphingoid base is selected from the group of sphinganines, sphingosines or phytosphingosines, most preferably phytosphingosines.

10. A method for preventing or treating an oral infection in a subject in need of such prevention or treatment, comprising administering to the oral cavity of said subject a composition releasing a sphingolipid at a rate of between 50 and 500 nmoles/min and allowing said subject to produce saliva while maintaining said composition in contact with the saliva in said oral cavity for a sufficient period of time to allow for the potentiation by said sphingolipid of a salivary antimicrobial peptide, thereby preventing or treating said oral infection.

11. Method according to claim 10, wherein said antimicrobial peptide is a histatin, preferably histatin 5.

12. Method according to claim 10 or 11, wherein said sphingolipid is a sphingoid base or a sphingomyelin, preferably a sphingoid base is selected from the group of sphinganines, sphingosines or phytosphingosines, most preferably phytosphingosines.

13. Method according to any one of claims 10-12, wherein said oral infection is selected from candidiasis, periodontitis, dental caries, and pulpitis.

14. Method according to any one of claims 10-13, wherein said period of time is between 15 seconds and 24 hours.

15. Use of a composition according to any one of claims 1-9 for potentiating the antimicrobial effect of salivary antimicrobial peptides in the saliva of a subject.

16. Use of a composition according to any one of claims 1-9 as an antimicrobial composition.

17. The combined use of a sphingolipid and a histatin for treating microbial infections.

18. Antimicrobial composition comprising a sphingolipid and a histatin.
